(19) Europäisches Patentamt
European Patent Office
Office européen des brevets

(11) Veröffentlichungsnummer: **0 027 960**
**B1**

(12) **EUROPÄISCHE PATENTSCHRIFT**

(45) Veröffentlichungstag der Patentschrift: **17.08.83**

(51) Int. Cl.³: **C 07 D 249/08**

(21) Anmeldenummer: **80106284.5**

(22) Anmeldetag: **16.10.80**

(54) **Verfahren zur Herstellung von 1,2,4-Triazol.**

(30) Priorität: **26.10.79 DE 2943265**

(43) Veröffentlichungstag der Anmeldung: **06.05.81 Patentblatt 81/18**

(45) Bekanntmachung des Hinweises auf die Patenterteilung: **17.08.83 Patentblatt 83/33**

(84) Benannte Vertragsstaaten: **AT BE CH DE FR GB IT LI NL**

(56) Entgegenhaltungen:
**DD-A-84 388**
**DE-A-2 802 491**
**US-A-2 763 661**
**JOHN WILEY & SONS, INC., New York — London, Vol. 7, 1961, R. C. ELDERFIELD »Heterocyclic compounds«, Seiten 425—445**
**I. Amer. Chem. Soc., Vol. 77, page 622 (1955)**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt/Main 80 (DE)**

(72) Erfinder: **Knorr, Harald, Dr., Hans-Fischer-Strasse 6, D-8906 Gersthofen (DE)**
Erfinder: **Maier, Thomas, Dr., Rauenthaler Weg 22, D-6000 Frankfurt am Main 71 (DE)**
Erfinder: **Mildenberger, Hilmar, Dr., Fasanenstrasse 24, D-6233 Kelkheim (Taunus) (DE)**
Erfinder: **Korbanka, Helmut, Dr., Birkenstrasse 24, D-8901 Adelsried (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Verfahren zur Herstellung von 1,2,4-Triazol

1,2,4-Triazol dient als Ausgangsmaterial für die Herstellung wertvoller fungizider Substanzen.

Die Verbindung kann in sehr guter Ausbeute durch Umsetzen von 1,3,5-Triazin mit Hydrazinhydrochlorid erhalten werden [C. J. Grundmann und A. Kreutzberger, J. Amer. Chem. Soc. 79, 2839 (1957); dto., J. Org. Chem. 21, 1037 (1956); US-A-2 800 486 (23. 07. 1957); C. A. 51, 18 009 a (1957)]. Dieses Verfahren ist jedoch wegen der hohen Kosten der Ausgangsmaterialien unwirtschaftlich und daher für die Synthese von 1,2,4-Triazol in technischen Mengen weniger geeignet. Zudem muß der sich bildende Chlorwasserstoff neutralisiert werden, wobei unerwünschte Salze in größeren Mengen anfallen.

Eine weitere Methode zur Synthese von 1,2,4-Triazol besteht in der Diazotierung von 3-Amino-1,2,4-trizaol, wobei Ausbeuten von ca. 75% erreicht werden [R. A. Henry und W. G. Finnegen, J. Amer. Chem. Soc. 76, 290 (1954)]. Nachteilig an diesem Verfahren ist die Notwendigkeit erhöhter sicherheitstechnischer Maßnahmen sowie die Gefahr der Bildung von Nitrosoverbindungen, die karzinogene Eigenschaften haben können.

Die Herstellung von 1,2,4-Triazol kann auch derart erfolgen, daß man N,N'-Diformylhydrazin über überschüssigem flüssigem Ammoniak im Autoklaven bei ca. 200° C umsetzt [C. Ainsworth und R. G. Jones, J. Amer. Chem. Soc. 77, 621 (1955)]. Man erhält hierbei das 1,2,4-Triazol in einer Ausbeute von 70 bis 80%.

Der Nachteil dieses Verfahrens besteht darin, daß zum einen unter hohem Druck im geschlossenen System gearbeitet werden muß, zum anderen, daß es erforderlich ist, einen sehr großen Überschuß an Ammoniak (20 Mol) und darüber hinaus auch noch in flüssiger Form einzusetzen, um die angegebenen Ausbeuten zu erzielen. Der Ammoniaküberschuß wird nach Beendigung der Reaktion abgelassen und muß im allgemeinen wieder kondensiert werden. Ein weiterer Nachteil dieses Verfahrens ist darin zu sehen, daß bei der Aufarbeitung eine Behandlung mit heißem Essigester erfolgt und auch dieses Lösungsmittel, wenn die Reaktion in technischen Mengen geführt werden soll, zu regenerieren und zu lagern ist.

In derselben Literaturstelle ist auch auf die Möglichkeit verwiesen, 1,2,4-Triazol aus Hydrazin, Formamid und Ammoniak mit gleichen Ausbeuten ebenfalls im Autoklaven herzustellen. Diesem Verfahren haften jedoch die oben angegebenen Mängel ebenfalls an, da der Unterschied in der Herstellung letztlich nur darin besteht, daß das N,N'-Diformylhydrazin nicht extra isoliert, sondern direkt weiter umgesetzt wird.

Aus der DE-A1-2 802 491 ist ein kontinuierliches Verfahren zur Synthese von 1,2,4-Triazol bekannt, bei welchem annähernd äquivalente Mengen Hydrazin bzw. -hydrat und Formamid mit Ammoniak bei 100 bis 250° C, gegebenenfalls auch drucklos in einer Reaktorkaskade unter Rückführung flüchtiger Produkte in die Kaskade umgesetzt werden. Nachteilig bei diesem Verfahren ist, daß das anfallende 1,2,4-Triazol nur eine Reinheit von ca. 95% besitzt.

Andere Verfahren zur Synthese von 1,2,4-Triazol sind wegen niedriger Ausbeuten von geringerem Interesse [G. Pellizzari, Gazz. chim. ital. 24, 222 (1894); dto. Ber. 27 R, 801 (1894); G. Pellizzari und C. Massa, Atti accad. Lincei (5) 10 I, 363 (1901); H. H. Strain, J. Amer. Chem. Soc. 49, 1996 (1927)].

Der vorliegenden Erfindung lag nun die Aufgabe zugrunde, ein Verfahren zur Herstellung von 1,2,4-Triazol zu finden, bei dem dieses in hohen Ausbeuten und mit hoher Reinheit anfällt, geringe Mengen Ammoniak benötigt werden und drucklos gearbeitet werden kann.

Überraschenderweise ist dies möglich, wenn man N,N'-Diformylhydrazin in Formamid bei erhöhten Temperaturen mit Ammoniak, welcher entweder direkt in der Reaktionsmischung von einer Ammoniak abspaltenden Verbindung geliefert oder auch in diese eingeleitet wird, umsetzt.

Die Erfindung betrifft somit ein verbessertes Verfahren zur Herstellung von 1,2,4-Triazol durch Cyclisierung von N,N'-Diformylhydrazin in Gegenwart von Ammoniak bei erhöhten Temperaturen, welches dadurch gekennzeichnet ist, daß man die benötigte Ammoniakmenge von 1 bis 5 Mol pro Mol N,N'-Diformylhydrazin entweder a) in situ aus Ammoniumcarbonat oder Ammoniumhydrogencarbonat durch Erhitzen auf 150 bis 250° C entstehen läßt, oder b) in das N,N'-Diformylhydrazin gasförmiges Ammoniak unter den gleichen Bedingungen einleitet, wobei in beiden Fällen drucklos und in der 2- bis 1[illegible]fachen Gewichtsmenge Formamid, bezogen auf N,N'-Diformylhydrazin, als Lösungsmittel gearbeitet und das gebildete Wasser laufend abdestilliert wird.

Es war nicht vorhersehbar, daß das 1,2,4-Triazol nach dieser Arbeitsweise überhaupt und dann auch noch in Ausbeuten von über 80% anfallen würde, nachdem entsprechend den Erkenntnissen von Ainsworth und Jones (loc. cit.) auch im vorliegenden Falle nur die Anwendung eines großen Ammoniaküberschusses in Verbindung mit hohem Druck Aussicht auf Erfolg versprach. Daß derartige Ausbeuten bereits bei Einsatz von (bezogen auf den Ammoniakgehalt) dem N,N'-Diformylhydrazin äquivalenten Mengen Ammoniumcarbonat bzw. -hydrogencarbonat oder gasförmigen Ammoniaks zu erreichen sind, war keineswegs zu erwarten. Es mußte nämlich damit gerechnet werden, daß das sich bildende Reaktionswasser unter diesen Verfahrensbedingungen sich nachteilig bei der Umsetzung auswirkt, weil — wie eigene Versuche gezeigt hatten — Diformylhy-

drazin beim Erhitzen mit Wasser zersetzt wird, bzw. mit wäßrigem Ammoniak nur ungenügend reagiert.

An Ammoniumcarbonat bzw. Ammoniumhydrogencarbonat wird so viel eingesetzt, daß pro Mol N,N'-Diformylhydrazin die äquivalente bis 5fach äquivalente Menge Ammoniak zur Verfügung steht. Bevorzugt arbeitet man mit 1 bis 3 Moläquivalent. Wird gasförmiger Ammoniak verwendet, so beträgt dessen Menge ebenfalls 1 bis 5, vorzugsweise jedoch 1 bis 2 Moläquivalent.

Die Umsetzung wird im Temperaturbereich von 150 bis 250, bevorzugt zwischen 160 und 200° C, vorgenommen.

Das N,N'-Diformylhydrazin kann nach der Vorschrift von C. Ainsworth und R. G. Jones [J. Amer. Chem. Soc. 77 (1955) 621] hergestellt werden. Es besitzt die Struktur

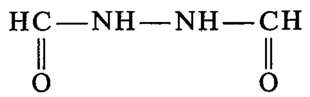

An die weiteren Ausgangsmaterialien, das sind das Lösungsmittel Formamid, welches in der 2- bis 10fachen Gewichtsmenge, bezogen auf N,N'-Diformylhydrazin, eingesetzt wird, sowie das Ammoniumcarbonat bzw. -hydrogencarbonat und das Ammoniakgas werden keine besonderen Anforderungen gestellt.

Die Synthese wird, wenn man nach Variante a mit in situ-Ammoniak arbeiten will, im einzelnen und bevorzugt so vorgenommen, daß man eine Mischung aus dem N,N'-Diformylhydrazin und dem jeweiligen Carbonat in Substanz oder als Suspension in auf 150 bis 250° C erhitztes Formamid portionsweise einbringt und jeweils abreagieren läßt. Eine weitere Möglichkeit besteht darin, eine N,N'-Diformylhydrazin-Ammoniumcarbonat-Formamid-Suspension durch erhitzte Zonen zu führen. Das bei der Reaktion frei werdende $CO_2$ entweicht zusammen mit dem Reaktionswasser und einer gewissen Menge Lösungsmittel, wobei die beiden letztgenannten in einem Kondensator abgeschieden werden. Zur Aufarbeitung des Reaktionsgemisches destilliert man zuerst das Formamid im Vakuum ab und erhält dann das 1,2,4-Triazol der Formel

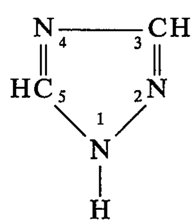

durch Vakuumdestillation.

Bei der Cyclisierungsmethode nach Variante b erfolgt die Umsetzung bevorzugt in zylindrischen Reaktoren oder Kolonnen. Sofern diskontinuierlich gearbeitet werden soll, legt man z. B. eine Lösung von N,N'-Diformylhydrazin in Formamid vor und leitet bei Temperaturen zwischen 150 und 250° C unter heftigem Bewegen der Reaktionsmischung, beispielsweise durch Rühren oder durch andere physikalische Operationen, z. B. Schwingungen, Ammoniakgas ein, während gleichzeitig das entstehende Reaktionswasser im Gemisch mit Formamid entweicht. Ein kontinuierliches Verfahren kann beispielsweise derart ausgeführt werden, daß man die Lösung von N,N'-Diformylhydrazin in Formamid über eine geheizte Füllkörperkolonne rieseln und gleichzeitig im Gleich- oder Gegenstrom Ammoniakgas einströmen läßt. Eine weitere Möglichkeit besteht darin, in einer gefluteten Kolonne zu arbeiten, der eine N,N'-Diformylhydrazin-Formamidmischung kontinuierlich zugeführt wird, während man das Reaktionsprodukt gleichzeitig kontinuierlich entnimmt.

Nachfolgende Beispiele sollen die Erfindung näher erläutern.

### Beispiel 1

In einem mit Destillationsaufsatz ausgerüsteten Rührkolben werden 280 ml Formamid vorgelegt und auf 180° C erhitzt. Man gibt hierzu in Portionen eine Mischung aus 2,4 Mol Ammoniumcarbonat (216,4 g) und 1,2 Mol (105,6 g) N,N'-Diformylhydrazin in der Weise, daß die Innentemperatur nicht unter 170° C fällt. Dabei wird Wasser und Formamid abdestilliert (102,6 g), während $CO_2$ und überschüssiges $NH_3$ entweichen. Nach Beendigung der Reaktion destilliert man zunächst im Wasserstrahlvakuum das Formamid und sodann das 1,2,4-Triazol ab. Ausbeute: 90,1% vom Schmp. 116° C (Lit. 120° C).

### Beispiel 2

Man arbeitet wie in Beispiel 1, verwendet jedoch nur die Hälfte = 108,2 g (1,2 Mol) Ammoniumcarbonat. Es destillieren 80,7 g Wasser und Formamid ab. Nach Beendigung der Reaktion wird wie angegeben aufgearbeitet. 89,3% 1,2,4-Triazol vom Schmp. 116° C.

### Beispiel 3

Nach der in Beispiel 1 beschriebenen Arbeitsweise werden 280 ml Formamid auf 170° C erhitzt, worauf man portionsweise eine Mischung aus 1,2 Mol N,N'-Diformylhydrazin und 1,2 Mol (94,8 g) Ammoniumhydrogencarbonat zufügt. Es destillieren 16,9 g Wasser und Formamid ab. Ausbeute: 89,9% 1,2,4-Triazol vom Schmp. 116° C.

### Beispiel 4

Man arbeitet wie in Beispiel 3 unter Einsatz von 189,6 g (2,4 Mol) Ammoniumhydrogencarbonat. Es destillieren 46,3 g Wasser und Formamid ab. Ausbeute: 78,6 g (95%) 1,2,4-Triazol vom Schmp. 116 bis 120° C.

Beispiel 5

Es wird wie in Beispiel 1 gearbeitet, man legt jedoch nur 100 ml Formamid vor und läßt so langsam eine Suspension der Reaktionspartner in 300 ml Formamid zulaufen, daß die Innentemperatur nicht unter 170° C absinkt. Ausbeute: 77 g 1,2,4-Triazol vom Schmp. 116 bis 120° C.

Beispiel 6

Durch eine mit Raschigringen gefüllte, beheizbare Kolonne von 0,5 m Länge und 4 cm Durchmesser läßt man bei einer Manteltemperatur von 180° C pro Stunde 85 g einer Lösung von N,N-Diformylhydrazin in Formamid (je Mol N,N'-Diformylhydrazin 375 g Formamid) hindurchtropfen. Gleichzeitig werden von unten in der Stunde 0,5 Mol Ammoniakgas eingeleitet. Das sich bei der Umsetzung bildende Wasser destilliert, zusammen mit etwas Formamid, über einen absteigenden Kühler am Kolonnenkopf laufend ab. Der Versuch wird nach sechs Stunden beendet. Nach dieser Zweit waren 20,4 g Kondensat abgeschieden worden. Aus der Reaktionsmischung destilliert man sodann im Vakuum das Formamid und schließlich das Triazol ab. Ausbeute an Triazol 71,4 g (86,3% d. Th.).

Beispiel 7

In einer beheizbaren 0,5 m langen Blasensäulenkolonne mit einem Durchmesser von 4 cm werden bei 170° C Innentemperatur 240 ml Formamid vorgelegt. Sodann wird eine Lösung von 105,6 g N,N'-Diformylhydrazin (1,2 Mol) in 240 ml Formamid innerhalb von 2½ Stunden von oben zugeführt und etwa 1,4 Mol Ammoniak von unten in der gleichen Zeit eingegast, wobei am Kolonnenkopf Wasser abdestilliert. Man läßt stets ebenso viel Reaktionslösung ablaufen, wie an Mischung zutropft. Nach Beendigung der Reaktion wird mit $N_2$ gespült, die Kolonne entleert und das Verfahrensprodukt einer Vakuumdestillation unterworfen, wobei zuerst das Formamid und schließlich das Triazol abdestilliert. Ausbeute: 74,7 g Triazol (90,3% d. Th.).

Beispiel 8

In der in Beispiel 7 beschriebenen Kolonne wird eine Lösung von 1,2 Mol N,N'-Diformylhydrazin in 240 ml Formamid bei 180° C Innentemperatur vorgelegt und innerhalb von 2 Stunden ca. 1,4 Mol Ammoniakgas von unten zugeführt, wobei Wasser im Gemisch mit Formamid abdestilliert (63,9 g). Nach Beendigung der Reaktion wird mit $N_2$ gespült und sodann der Reaktorinhalt abgelassen. Die Reaktionsmischung wird wie beschrieben aufgearbeitet. Ausbeute: 78,9 g Triazol (95,3% d. Th.).

Beispiel 9

Man arbeitet wie in Beispiel 8 und gast 2,6 Mol Ammoniak innerhalb von 4 Stunden ein. Es werden 79,4 g (95,7% d. Th.) Triazol erhalten.

Beispiel 10

In einem mit einem Rührwerk und diversen Flügelrührern versehenen, beheizbaren, zylindrischen Reaktor von 10 Litern Inhalt wird bei 170° C Innentemperatur eine Lösung von 880 g N,N'-Diformylhydrazin (10 Mol) in 3400 g Formamid heftig gerührt. Gleichzeitig leitet man innerhalb von 3,5 Stunden von unten 14,1 Mol Ammoniakgas ein, wobei Wasser im Gemisch mit Formamid abdestilliert (453,7 g Destillat). Nachdem die Reaktion beendet ist, spült man mit $N_2$ und entleert den Reaktor. Die Reaktionsmischung wird im Vakuum destilliert, wobei zuerst eine geringe Menge einer Mischung aus Wasser und Formamid, dann Formamid und schließlich Triazol übergeht. Ausbeute an Triazol 634 g (91,9% d. Th.).

Beispiel 11

In der in Beispiel 1 beschriebenen Apparatur wird eine Mischung aus 423 g (4,8 Mol) N,N'-Diformylhydrazin und 720 g Formamid vorgelegt und auf 170 bis 175°C erhitzt. Man fügt nun portionsweise innerhalb von 6 Stunden 758 g (9,6 Mol) $NH_4HCO_3$ so zu, daß die Innentemperatur nicht unter 165°C fällt. Es destilliert ein Gemisch aus Wasser und Formamid ab, während $CO_2$ und überschüssiges $NH_3$ entweichen. Man arbeitet wie in Beispiel 1 angegeben auf und destilliert schließlich das Triazol im Wasserstrahlvakuum ab. Ausbeute 298 g (90%), Schmp. 116 bis 120° C.

**Patentansprüche**

1. Verfahren zur Herstellung von 1,2,4-Triazol durch Cyclisierung von N,N'-Diformylhydrazin in Gegenwart von Ammoniak bei erhöhten Temperaturen, dadurch gekennzeichnet, daß man die benötigte Ammoniakmenge von 1 bis 5 Mol pro Mol N,N'-Diformylhydrazin entweder a) in situ aus Ammoniumcarbonat oder Ammoniumhydrogencarbonat durch Erhitzen auf 150 bis 250°C entstehen läßt, oder b) in das N,N'-Diformylhydrazin gasförmiges Ammoniak unter den gleichen Bedingungen einleitet, wobei in beiden Fällen drucklos und in der 2- bis 10fachen Gewichtsmenge Formamid, bezogen auf N,N'-Diformylhydrazin, als Lösungsmittel gearbeitet und das gebildete Wasser laufend abdestilliert wird.

2. Verfahren nach Anspruch 1, Variante a, dadurch gekennzeichnet, daß man entweder $\alpha$) eine Mischung aus N,N'-Diformylhydrazin und

Ammoniumcarbonat bzw. Ammoniumhydrogencarbonat oder eine Suspension dieser Mischung in Formamid, in auf 150 bis 250°C erhitztes Formamid einträgt, oder β) das Ammoniumcarbonat oder -hydrogencarbonat, gegebenenfalls auch als Suspension in Formamid, zu einer auf 150 bis 250°C erhitzten Mischung aus N,N'-Diformylhydrazin und Formamid gibt.

## Claims

1. A process for the preparation of 1,2,4-triazole by cyclization of N,N'-diformylhydrazine in the presence of ammonia at elevated temperatures, which comprises supplying the ammonia required in an amount of from 1 to 5 moles per mole of N,N-diformylhydrazine either a) by forming it in situ from ammonium carbonate or ammonium hydrogen carbonate with heating to 150 to 250°C, or b) introducing gaseous ammonia into the N,N'-diformylhydrazine under the same conditions, while operating in both cases without pressure and in a 2- to 10-fold weight amount (relative to N,N'-diformylhydrazine) of formamide as solvent, and constantly distilling off the water formed.

2. The process of Claim 1, variant a), which comprises α) either introducing a mixture of N,N'-diformylhydrazine and ammonium carbonate or ammonium hydrogen carbonate, or a suspension of this mixture in formamide, into formamide heated to 150 to 250°C, or β) adding the ammonium carbonate or ammonium hydrogen carbonate, optionally in the form of a suspension in formamide, to a mixture of N,N'-diformylhydrazine and formamide heated to 150 to 250°C.

## Revendications

1. Procédé de préparation du triazole-1,2,4 par cyclisation de la N,N'-diformylhydrazine en présence d'ammoniac à température élevée, procédé caractérisé en ce que la quantité d'ammoniac nécessaire, qui est de 1 à 5 moles par mole de N,N'-diformylhydrazine, est soit (a) créée in situ à partir de carbonate d'ammonium ou d'hydrogénocarbonate d'ammonium par chauffage à une température de 150 à 250°C, soit (b) introduite à l'état gazeux dans la N,N'-diformylhydrazine, dans les mêmes conditions, et en ce que, dans les deux cas, on opère sans pression et dans du formamide, comme solvant, utilisé en un poids représentant de 2 à 10 fois celui de la N,N'-diformylhydrazine, et l'eau formée est chassée continuellement par distillation.

2. Procédé selon la revendication 1, variante (a), caractérisé en ce que ou bien (α) on introduit dans du formamide chauffé à une température de 150 à 250°C un mélange de N,N'-diformylhydrazine et de carbonate d'ammonium ou d'hydrogénocarbonate d'ammonium ou une suspension de ce mélange dans du formamide, ou bien (β) on ajoute le carbonate ou l'hydrogénocarbonate d'ammonium, éventuellement sous la forme d'une suspension dans du formamide, à un mélange de N,N'-diformylhydrazine et de formamide qui est chauffé à une température de 150 à 250°C.